# EUROPEAN PATENT APPLICATION

(11) **EP 3 646 851 A2**
(43) Date of publication of application: **06.05.2020**
(21) Application number: 18824818.1
(22) Date of filing: 25.06.2018
(51) Int. Cl.: A61K 8/99, A61Q 19/00, A61Q 19/08, A61Q 17/04

(54) **ANTI-AGING COMPOSITION COMPRISING LACTIC ACID BACTERIA-DERIVED EXTRACELLULAR VESICLE**

(30) Priority: 29.06.2017 KR 20170082464
(71) Applicant: Amorepacific Corporation, Seoul 04386 (KR)
(72) Inventor: KIM, Wanil, Yongin-si Gyeonggi-do 17074 (KR); CHO, Eun-Gyung, Yongin-si Gyeonggi-do 17074 (KR); LEE, Tae Ryong, Yongin-si Gyeonggi-do 17074 (KR)
(74) Representative: Delorme, Nicolas
(86) International application number: PCT/KR2018/007166
(87) International publication number: WO 2019/004673

(57) **Abstract**

Disclosed in the specification is an anti-aging composition comprising, as an effective ingredient, extracellular vesicles that are complex physiologically active substances derived from lactic acid bacteria. The extracellular vesicles may be exosome-like vesicles with a diameter of 20 to 200 nm and the composition inhibits the expression of MMP-1 protein, thereby providing the effects of improving skin wrinkles, increasing skin elasticity, suppressing collagen reduction, and preventing ultraviolet light-induced skin damage.

## Description

### [Technical Field]

The present disclosure relates to an anti-aging composition comprising as an effective ingredient an extracellular vesicle derived from a lactic acid bacteria.

### [Background Art]

Matrix metalloproteinase (hereinafter referred to as "MMP") is a zinc -containing endopeptidase enzyme capable of decomposing macromolecules such as collagen, proteoglycan and gelatin, and is largely classified into collagenase, gelatinase, stromelysin and membrane type MMP. Since MMP induces skin aging, if it is inhibited, an effect of wrinkle treatment and prevention can be expected. In addition, MMP promotes angiogenesis, cancer invasion and metastasis by decomposition of basement membrane.

Collagen present in the dermis of the skin accounts for about 70-80% of the dry weight of the skin, and is known to control skin elasticity with elastin, which is an elastic fiber. MMP plays an important role in the reduction of collagen, which is involved in the decomposition of extracellular matrix and basement membrane. It has been reported that the activity of the enzyme is increased by aging or ultraviolet rays, and that the inhibition of MMP reduced skin aging phenomena, such as skin thickening and wrinkle formation, induced by aging or ultraviolet rays (Inomata S et al., J. Invest. Dermatol., 120 (1): 128-134, 2003).

Meanwhile, most animal cells have the ability to secrete extracellular vesicles of intracellular origin with various sizes and components, and these extracellular vesicles are found in all biological fluids including blood, urine, saliva, and cell culture fluids (Loyer X, Vion AC, Tedgui A, Boulanger CM. Microvesicles as cell-cell messengers in cardiovascular diseases. Circ Res 2014; 114: 345-53; Ohno S, Ishikawa A, Kuroda M. Roles of exosomes and microvesicles in disease pathogenesis. Adv Drug Deliv Rev 2013; 65: 398-401).

The extracellular vesicles are small vesicles having a membrane structure, the diameter ranging from about 20 nm to about 5 µm, and are heterogeneous in their size and composition. The extracellular vesicles include many different types including exosomes (about 30-100 nm), ectosomes, microvesicles (about 100-1,000 nm), microparticles, apoptotic bodies (about 1-5 µm) and others.

The different types of extracellular vesicles are distinguished according to their origin, diameter, density in sucrose, shape, sedimentation rate, lipid composition, protein marker or secretion mode (whether the secretion is by a signal (inducible) or spontaneous (constitutive)). For example, microvesicles are membrane vesicles with irregular shapes of about 100 to 1,000 nm and known to germinate toward the outside of the plasma (originate from the plasma membrane) and to contain markers, including integrin, selectin, CD40 ligand, etc., and phospholipids, including phosphatidylserine. Meanwhile, exosomes are the smallest membrane vesicles having a cup shape of about 30 to 100 nm (<200 nm), and are known to germinate from the inside of late endosomes (originating from endosomes) and to generally contain markers, including CD63, tetraspanin of CD9, TSG101 and ESCRT, and phospholipids including cholesterol, sphingomyelin, ceramide, and phosphatidylserine.

These extracellular vesicles reflect the state of secretory cells (donor cells), and exhibit various biological activities depending on the cells from which the vesicles are secreted. The extracellular vesicles play an important role in cell-to-cell communication by transferring genetic material and proteins between cells.

Prokaryotic or eukaryotic cells are also known to secrete extracellular vesicles (Camussi, G., Deregibus, M. C., Bruno, S., Cantaluppi, V., & Biancone, L. (2010). Exosomes/microvesicles as a mechanism of cell-to-cell communication. Kidney International, 78(9), 838-848; Bang, Claudia, and Thomas Thum. "Exosomes: New players in cell-cell communication." The International Journal of Biochemistry & Cell Biology 44.11 (2012): 2060-2064; Kim, D. K., Lee, J., Simpson, R. J., Lotvall, J., & Gho, Y. S. (2015, April), EVpedia: A community web resource for prokaryotic and eukaryotic extracellular vesicles research. Seminars in Cell & Developmental Biology (Vol. 40, pp. 4-7). Academic Press; Kim, J. H., Lee, J., Park, J., & Gho, Y. S. (2015, April). Gram-negative and Gram-positive bacterial extracellular vesicles. Seminars in Cell & Developmental Biology (Vol. 40, pp. 97-104). Academic Press).

Lactic acid bacterium, which is a representative microorganism useful for human body, is a bacterium that decomposes sugars such as glucose or lactose to produce organic acids such as lactic acid and acetic acid, and is used to manufacture fermented foods such as fermented milk, cheese and butter through a fermentation process to produce organic acids from sugars. However, the anti-aging effect of extracellular vesicles, which are complex physiologically active substances derived from lactic acid bacteria, has not been disclosed. The related prior art includes Korean Patent Laid-Open No. 10-2011-0025603.

### [Technical Problem]

In one aspect, the present disclosure aims to provide an anti-aging composition comprising as an effective ingredient an extracellular vesicle, which is a complex physiologically active substance derived from a lactic acid bacterium.

In another aspect, the present disclosure aims to provide a method for producing an anti-aging composition comprising as an effective ingredient an extracellular vesicle derived from a lactic acid bacterium.

### [Solution to Problem]

In one aspect, the technology disclosed herein provides an anti-aging composition comprising as an active ingredient an extracellular vesicle derived from a lactic acid bacterium.

In one exemplary embodiment, the lactic acid bacterium may be at least one selected from the group consisting of *Bifidobacterium spp., Latobacillus spp., Streptocuccus spp., Leuconostoc spp., Pediococcus spp.* and *Lactococcus spp.* strains.

In one exemplary embodiment, the lactic acid bacterium may be a strain of *Latobacillus spp.*

In one exemplary embodiment, the strain of *Latobacillus spp.* may be *Lactobacillus* plantarum.

In an exemplary embodiment, the extracellular vesicle may be isolated from a lactic acid bacteria culture solution.

In an exemplary embodiment, the extracellular vesicle may be an exosome-like vesicle.

In an exemplary embodiment, the exosome-like vesicle may have a diameter of 20 to 200 nm.

In an exemplary embodiment, the extracellular vesicle may be precipitated by an ultracentrifugation of 100,000 x g or higher of a lactic acid bacteria culture.

In an exemplary embodiment, the composition may be one that inhibits the expression of the MMP-1 enzyme.

In an exemplary embodiment, the composition may be one that inhibits the expression of MMP-1 enzyme induced by ultraviolet light.

In an exemplary embodiment, the composition may have at least one properties selected from the group consisting of (a) improving skin wrinkles; (b) increasing skin elasticity; (c) suppressing collagen reduction; and (d) preventing ultraviolet light-induced skin damage.

In an exemplary embodiment, the composition may be an external skin application composition.

In an exemplary embodiment, the composition may be a cosmetic composition.

### [Advantageous Effects of Invention]

In one aspect, the technology disclosed in the present specification has provides an anti-aging composition comprising as an effective ingredient an extracellular vesicle, which is a complex physiologically active substance derived from a lactic acid bacterium.

In another aspect, the technology disclosed in the present specification provides a method for producing an anti-aging composition comprising as an effective ingredient an extracellular vesicle derived from a lactic acid bacterium.

### [Brief Description of Drawings]

FIG. 1 shows the steps of the process of separating extracellular vesicles from lactic acid bacteria according to one embodiment of the present specification.
FIG. 2A shows a transmission electron microscope image of lactic acid bacteria-derived extracellular vesicles according to one test example of the present specification.
FIG. 2B shows the result of the measurement of the extracellular vesicle size derived from lactic acid bacteria according to one test example of the present specification.
FIG. 3 shows the result of the measurement of the effect on cell proliferation and cytotoxicity of dermal fibroblasts treated with lactic acid bacteria-derived extracellular vesicles according to a test example of the present specification (**, p <0.01).
FIG. 4 shows the result of the measurement of the effect on the expression of MMP-1 protein of lactic acid bacteria-derived extracellular vesicles treated with dermal fibroblasts according to a test example of the present specification (**, p <0.01).
FIG. 5 shows the results of comparing the effect on cytotoxicity of lactic acid bacteria-derived extracellular vesicles with that of lactic acid bacteria cells according to one test example of the present specification.

### [Description of Embodiments]

Hereinafter, the present disclosure will be described in detail.

In one aspect, the present disclosure provides an anti-aging composition comprising as an active ingredient an extracellular vesicle derived from lactic acid bacteria.

In another aspect, the present disclosure provides an anti-aging method comprising applying an effective amount of an extracellular vesicle derived from lactic acid bacteria for anti-aging to a subject in need thereof.

In another aspect, the present disclosure provides an extracellular vesicle derived from lactic acid bacteria for anti-aging of a subject.

In another aspect, the present disclosure provides a non-therapeutic use of an extracellular vesicle derived from lactic acid bacteria for anti-aging of a subject.

In another aspect, the present disclosure provides a use of an extracellular vesicle derived from lactic acid bacteria in manufacturing a composition for anti-aging of a subject.

In an exemplary embodiment, the ant-aging may be skin ant-aging.

In an exemplary embodiment, the lactic acid bacteria-derived extracellular vesicle may be applied or administered to or coated on a subject in the form of a pharmaceutical composition, an external skin application composition, a cosmetic composition or a food composition.

In an exemplary embodiment, the lactic acid bacteria-derived extracellular vesicle may be applied to a skin or scalp of a subject.

As used herein, the term "active ingredient" refers to a component that can exhibit the desired activity alone or with a carrier, etc. which is inactive itself.

In the present specification, the lactic acid bacteria can be used without limitation, either as an isolated strain or a variety of commercially available lactic acid bacteria.

In an exemplary embodiment, the lactic acid bacteria may be one obtained from green tea.

In one exemplary embodiment, the lactic acid bacteria may be at least one selected from the group consisting of *Bifidobacterium* spp., *Latobacillus* spp., *Streptocuccus* spp., *Leuconostoc* spp., *Pediococcus* spp. and *Lactococcus* spp., and a strain of the *Lactobacillus* genus may be preferable in terms of increasing the yield of extracellular vesicles including exosome.

In one exemplary embodiment, the lactic acid bacteria may be at least one selected from the group consisting of *Bifidobacterium breve* (*B. breve*), *Bifidobacterium longum (B. longum), Bifidobacterium infantis (B. infantis), Lactobacillus bulgaricus (L. bulgaricus), Lactobacillus reuteri (L. reuteri), Lactobacillus rhamnosus (L. rhamnosus), Lactobacillus plantarum (L. plantarum), Lactobacillus sakei (L. sakei), Lactobacillus acidophilus (L. acidophilus), Streptococcus thermophilus (S. thermophilus), Streptococcus faecium (S. faecium), Streptococcus faecalis (S. faecalis), Leuconostoc mesenteroides (L. mesenteroides), Pediococcus cerevisiae* (*P. cerevisiae*), and *Lactococcus lactis (L. lactis*), and *Lactobacillus plantarum* may be the most preferable in terms of increasing the yield of extracellular vesicles including exosome. *Lactobacillus plantarum* increases the yield of exosomes.

In an exemplary embodiment, the lactic acid bacteria may be *Lactobacillus plantarum* APsulloc 331262 (KCCM 11179P) .

As used herein, the term "an extracellular vesicle" refers to a vesicle secreted from cells and released into the extracellular space. The inside and the outside of the vesicle are distinguished by a lipid bilayer, and the vesicle has plasma membrane lipids, plasma membrane proteins, nucleic acids, and cytoplasmic components that can be used to indirectly understand the nature and state of cells. The extracellular vesicle can bind to other cells or tissues to deliver membrane components, mRNAs, miRNAs, proteins (growth hormone, cytokine, etc.), and transfer these substances to the recipient cells to act as extracellular transporters mediating cell-to-cell communication.

As used herein, the term "exosome-like vesicle" refers to a nanosized extracellular vesicle and the term is used in the broadest concept, including not only a nanosized exosome but also a vesicle similar in a nanosized vesicular structure and a composition with the exosome.

In an exemplary embodiment, the extracellular vesicle may be isolated in a lactic acid bacteria culture. The isolated extracellular vesicle may be physically separated, wholly or partially, from the existing tissues or cells.

In an exemplary embodiment, the extracellular vesicle may have a diameter of 20 to 500 nm. In another aspect, the extracellular vesicle may have a diameter of at least 20 nm, at least 30 nm, at least 40 nm, at least 50 nm, at least 60 nm, at least 70 nm, at least 80 nm, at least 90 nm or at least 100 nm, or 500 nm or less, 450 nm or less, 400 nm or less, 350 nm or less, 300 nm or less, 250 nm or less, 200 nm or less, 150 nm or less or 100 nm or less.

In an exemplary embodiment, the extracellular vesicle may be an exosome-like vesicle.

In an exemplary embodiment, the exosome-like vesicle may have a diameter of 20 to 200 nm. In another aspect, the exosome-like vesicle may have a diameter of at least 20 nm, at least 30 nm, at least 40 nm, at least 50 nm, at least 60 nm, at least 70 nm, at least 80 nm, at least 90 nm, or at least 100 nm, or 200 nm or less, 190 nm or less, 180 nm or less, 170 nm or less, 160 or less, 150 nm or less, 140 nm or less, 130 nm or less, 120 nm or less, 110 nm or less or 100 nm or less.

In an exemplary embodiment, the extracellular vesicle may be an exosome.

In an exemplary embodiment, the extracellular vesicle may be one that precipitates in ultracentrifugation of lactic acid bacteria culture at 100,000 x g or higher, specifically 100,000 to 200,000 x g, or 100,000 to 150,000 x g, or 150,000 to 200,000 x g.

In an exemplary embodiment, the extracellular vesicle may be one in which the membrane component is chemically or physically modified, for example, to efficiently perform a desired function in a target cell. For example, the membrane component of the extracellular vesicle may be modified by a chemical method using a thiol group (-SH) or an amine group (-NH₂), or the membrane component of the extracellular vesicle may be modified chemically by a chemical bonding of a target inducing substance, a cell membrane fusion substance or polyethylene glycol to the extracellular vesicle

In an exemplary embodiment, the extracellular vesicle may be separated by using at least one method selected from the group consisting of centrifugation, ultracentrifugation, differential centrifugation, equilibrium density centrifugation, density gradient, filtration, dialysis and free-flow electrophoresis but not limited thereto.

Density gradient is the method that is most commonly used in distinguishing substances of different densities. Specific examples of this method include those performed by using density gradient materials such as ficoll, glycerol, sucrose, cesium chloride and iodixanol but are not limited thereto. In one aspect, density gradient can be used with ultracentrifugation and the like. In another aspect, gel filtration or ultrafiltration may be used to screen extracellular vesicles. In another aspect, dialysis may be used instead of filtration to remove molecules of a small size. In another aspect, free-flow electrophoresis may be used.

In an exemplary embodiment, the extracellular vesicle may be prepared by a method comprising: (1) centrifuging the culture of the lactic acid bacteria to obtain a supernatant; (2) filtering the obtained supernatant; and (3) ultra-centrifugating the obtained filtrate to obtain a precipitate, wherein the lactic acid bacteria-derived extracellular vesicle exhibits effective activity.

In an exemplary embodiment, the centrifugation in (1) above may be performed at 1,000 to 20,000 x g, or 1,500 to 20,000 x g, or 1,500 to 15,000 x g, or 1,500 to 10,000 x g for 30 to 60 minutes. At this time, the centrifugation may be performed stepwise by changing the speed or time. For example, the centrifugation may be performed at a low speed of 1,500 to 2,000 x g to separate and remove the lactic acid bacteria strain from the culture, and then at a high speed of 10,000 to 20,000 x g to further remove the cells or cell-related debris and residues, etc.

In an exemplary embodiment, the filtration in (2) above be performed with a filter having a size of 0.3 to 0.5 µm. The purity of the centrifuged culture solution may be increased through the filtration.

In an exemplary embodiment, the ultracentrifugation in (3) above may be performed at 100,000 x g or higher, specifically 100,000 to 200,000 x g, or 100,000 to 150,000 x g, or 150,000 to 200,000 x g for 1 to 5 hours.

In an exemplary embodiment, after (3) and (4) above, suspending the obtained precipitate may be further included.

As used herein, the term "anti-aging" means a use for preventing, delaying, and/or improving aging caused by internal factors, including genetic factors, and external factors, including ultraviolet rays, etc. For example, "anti-aging" means preventing and delaying, or improving and alleviating skin changes due to aging, such as skin thickness increase, skin barrier damage, skin elasticity reduction, wrinkle formation, increase in the depth or number of wrinkles, skin dryness, skin roughness and ultraviolet light-induced skin damage.

In an exemplary embodiment, the composition may be an anti-aging composition against photoaging.

In an exemplary embodiment, the composition may be an anti-aging composition that improves skin wrinkles or increases skin elasticity. Specifically, the composition has the effect of preventing or blocking the generation of wrinkles by delaying the wrinkle generation timing as much as possible, or alleviating or improving wrinkles that have already been generated. In addition, the composition has the effect of preventing or blocking skin elasticity reduction, or increasing or improving skin elasticity.

In an exemplary embodiment, the composition may inhibit the expression of collagenase, or reduce the activity of collagenase that is minimally expressed by the composition of the present specification. In another aspect, the composition may also have specific effects on substances such as up-stream enzymes or proteins that reduce the expression or activity of collagenase.

MMP-1 is a proteolytic enzyme in the skin that degrades collagen type I, which is an extracellular matrix, and functions as an important protein for intercellular morphogenesis, wound healing, tissue regeneration and reorganization. However, when a person is aged or exposed to ultraviolet rays, the production of MMP-1 is increased in the body and MMP-1 binds to collagen and then cuts collagen to cause skin aging such as wrinkle generation and reduction of elasticity. The composition according to the present disclosure has the effect of preventing, delaying and/or improving skin aging by inhibiting the activity of MMP-1, which is a collagenase.

In an exemplary embodiment, the composition may be a lyophilized formulation contained in a ready-to-use sealed package or in a packaging container.

The present disclosure also provides an anti-aging kit, comprising a composition comprising as an active ingredient a lactic acid bacteria-derived extracellular vesicle and having a lyophilized formulation; and sterile water or purified water. The kit may be a kit contained in a sealed packaging material or in a packaging container so that it can be used readily (ready-to-use).

In an exemplary embodiment, the composition may be an external skin application composition, wherein the external skin application composition refers to anything that may be externally applied to a skin and may comprise various formulations of pharmaceuticals. For example, the composition may be in the form of ointments, lotions, gels, creams, sprays, suspensions, emulsions, patches, etc., but is not limited thereto.

In an exemplary embodiment, the composition may be a cosmetic composition.

The cosmetic composition may further contain, in addition to lactic acid bacteria-derived extracellular vesicles, a functional additive and components included in a general cosmetic composition. The functional additive may include a component selected from the group containing water-soluble vitamins, oil-soluble vitamins, polymer peptides, polymeric polysaccharides, sphingolipids and seaweed extracts. Other ingredients that may be included in the composition include an oil component, a moisturizing agent, an emollient, a surfactant, an organic or inorganic pigment, an organic powder, an ultraviolet absorber, a preservative, a bactericide, an antioxidant, a plant extract, a pH regulator, an alcohol, a coloring agent, a flavoring agent, a blood circulation accelerator, a cooling agent, an antiperspirant agent and purified water.

The formulation of the cosmetic compositions is not particularly limited and may be appropriately selected according to the purpose. For example, the cosmetic composition may be prepared in at least one formulation selected from the group containing skin lotion, skin softener, skin toner, astringent, lotion, milky lotion, moisturizing lotion, nutrition lotion, massage cream, nutritional cream, moisturizing cream, hand cream, foundation, essence, nutrition essence, pack, soap, cleansing foam, cleansing lotion, cleansing cream, body lotion and body cleanser, but is not limited thereto.

When the formulation of the present disclosure is a paste, cream or gel, animal fiber, plant fiber, wax, paraffin, starch, tragacanth, cellulose derivative, polyethylene glycol, silicone, bentonite, silica, talc or zinc oxide may be used as a carrier component.

When the formulation of the present disclosure is a powder or a spray, lactose, talc, silica, aluminum hydroxide, calcium silicate or polyamide powder may be used as a carrier component. In the case of a spray, in particular, a propellant such as chlorofluorohydrocarbon, propane or dimethyl ether may be further included.

When the formulation of the present disclosure is a solution or an, a solvent, a solvating agent or an emulsifier is used as a carrier component, and examples thereof include water, ethanol, isopropanol, ethyl carbonate, ethyl acetate, benzyl alcohol, benzyl benzoate, propylene glycol, 1,3-butyl glycol oil, glycerol aliphatic ester, polyethylene glycol or sorbitan fatty acid ester.

When the formulation of the present disclosure is a suspension, a liquid diluent such as water, ethanol and propylene glycol; a suspending agent such as ethoxylated isostearyl alcohol, polyoxyethylene sorbitol ester and polyoxyethylene sorbitan ester; microlite cellulose, aluminum metahydroxide, bentonite, agar, tragacanth, etc. may be used as a carrier component.

When the formulation of the present disclosure is an surfactant-containing cleansing, aliphatic alcohol sulfate, aliphatic alcohol ether sulfate, sulfosuccinate monoester, isethionate, imidazolinium derivative, methyltaurate, sarcosinate, fatty acid amide ether sulfates, alkylamido betaines, aliphatic alcohols, fatty acid glycerides, fatty acid diethanolamides, vegetable oils, linolenic derivatives or ethoxylated glycerol fatty acid esters may be used as a carrier component.

### [Examples]

Hereinafter, the present disclosure will be described in more detail with reference to Examples. It would be obvious to one of ordinary skill in the art that these Examples are merely illustrative of the present disclosure and that the scope of the present disclosure is not construed as being limited by these Examples.

### Example 1. Separation of Extracellular Vesicles Derived from Lactic Acid Bacteria

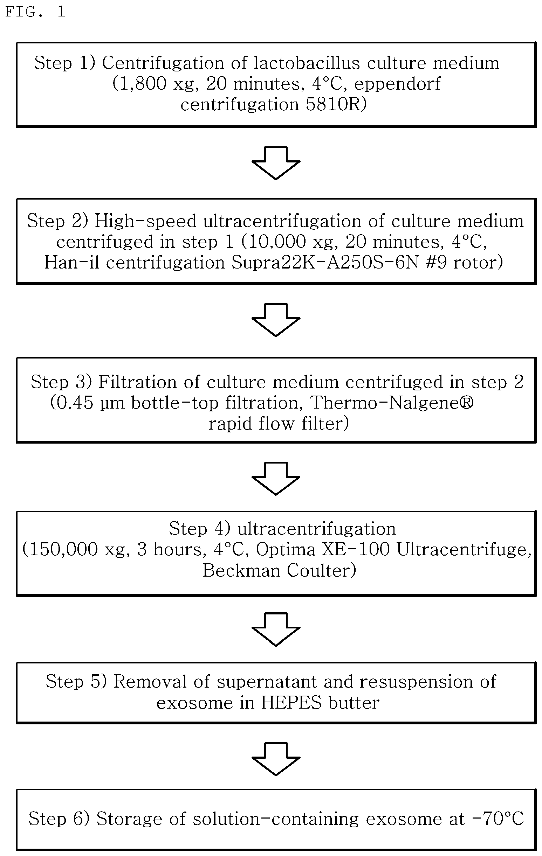

In this Example, a *Lactobacillus* strain obtained from green tea was used as lactic acid bacteria, and extracellular vesicles containing exosome were isolated therefore. More specifically, *Lactobacillus plantarum* APsulloc 331261 (Accession No.: KCCM11179P) was used to prepare extracellular vesicles derived from lactic acid bacteria.
(Step 1) The culture solution of the cultured *Lactobacillus* strain was centrifuged at 1,800 x g at 4°C for 20 minutes to separate and remove the *Lactobacillus* strain from the culture solution. In other words, the cells and the supernatant were separated through a low-speed centrifugation process.
(Step 2) The culture solution centrifuged in Step 1 was centrifuged at 10,000 x g for 20 minutes at 4°C by using a high-speed centrifuge to further remove the cells or cell-related debris and residues.
(Step 3) The culture solution centrifuged in Step 2 was filtered through a 0.45 µm bottle-top filter to increase the purity of the culture solution.
(Step 4) The extracellular vesicles containing exosome were separated by ultracentrifugation at 150,000 x g for 3 hours at 4°C.
(Step 5) After removing the supernatant obtained in Step 4 through the ultracentrifugation, the remaining pellet of the extracellular vesicle containing exosome was resuspended in a buffer solution (HEPES buffer).
(Step 6) The extracellular vesicle (EV) containing exosomes resuspended in the buffer solution in Step 5 was stored at -70°C and used in the following test (see FIG. 1) .

### Comparative Example 1. Preparation of Lactic Acid Bacterial Cells

Lactic acid bacterial cells were prepared by using the *Lactobacillus plantarum* APsulloc 331261 (Accession No.: KCCM11179P) strain used in Example 1 and used in the following test. In other words, the lactic acid bacterial cells used in the present Comparative Example mean the intact cells that have not undergone a process of separating the extracellular vesicles.

### Test Example 1. Verification of Extracellular Vesicle (EV) Derived from Lactic Acid Bacteria

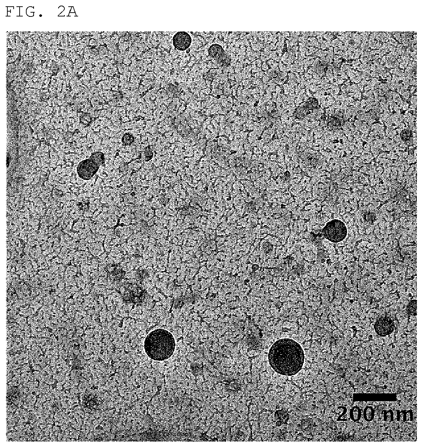

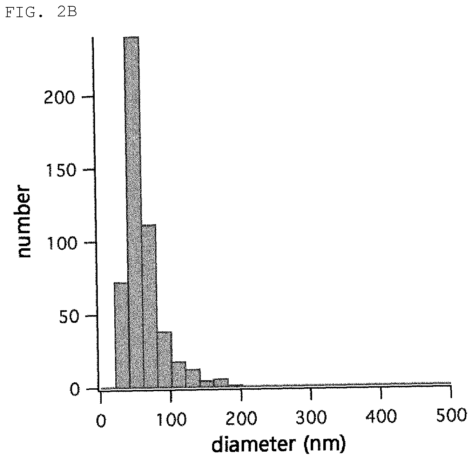

The shape and size of the extracellular vesicles from the lactic acid bacteria isolated in Example 1 were analyzed by a transmission electron microscope and dynamic light scattering (DLS), and the result showed that extracellular vesicles were generally circular and had a diameter of about 20 to 200 nm (see FIGS. 2A and 2B). In addition, the result showed that the isolated extracellular vesicles had a diameter of about 20 to 200 nm and an average diameter of about 50 to 60 nm, more specifically, 55.46 nm.

### Test Example 2. Effect on Cell Proliferation and Cytotoxicity

The extracellular vesicles (0.1 to 10 µg / ml) derived from the lactic acid bacteria isolated in Example 1 were treated with human fibroblast (Hs68) to verify the effect on cell proliferation and cytotoxicity.

Specifically, human fibroblast Hs68 was cultured in a CO₂ incubator, treated with the extracellular vesicles isolated from green tea-derived lactic acid bacteria in Example 1 at 0.1 µg/ml, 1 µg/ml, and 10 µg/ml for 48 hours under the serum-free conditions. A positive control was treated with 5 ng/ml of TGF-β. Then, the cells were further cultured for 2 hours after exchanging the medium with the one containing 5% CCK-8 solution. Finally, the absorbance at 450 nm wavelength was measured to calculate the cell proliferation and cytotoxicity in comparison with the control group.

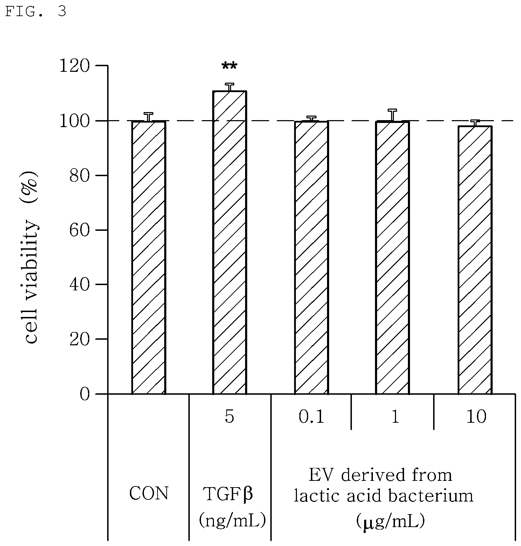

As shown in FIG. 3, the result verified that the extracellular vesicles derived from the lactic acid bacteria did not affect the cell proliferation and were stable in the absence of cytotoxicity.

### Test Example 3. MMP -1 Protein Expression

The extracellular vesicles (0.1-10 µg/ml) derived from the lactic acid bacteria isolated in Example 1 were treated with human fibroblast (Hs68) to verify the effect on the expression of MMP-1 protein, which is secreted extracellularly.

Specifically, human fibroblast Hs68 was cultured in a 6-well plate at 1.8 × 10⁵ cells/2 mL per well for 24 hours. After substituting a serum-free medium, the cells were further cultured for 6 hours, and then stimulated with UVB 20 mJ/cm². The cells were then treated with the extracellular vesicles derived from the green tea-derived lactic acid bacteria isolated in Example 1 at 0.1 µg/ml and 1 µg/ml, and the positive control was treated with 1 µM of retinoic acid (RA). After 48 hours of 5% CCK-8 solution treatment, the culture solutions were collected and the amount of MMP-1 was measured by ELISA. In addition, in order to correct the difference in the amount of the cells between the groups, the MMP-1 ELISA results were normalized to the cell proliferation and cytotoxicity results described above.

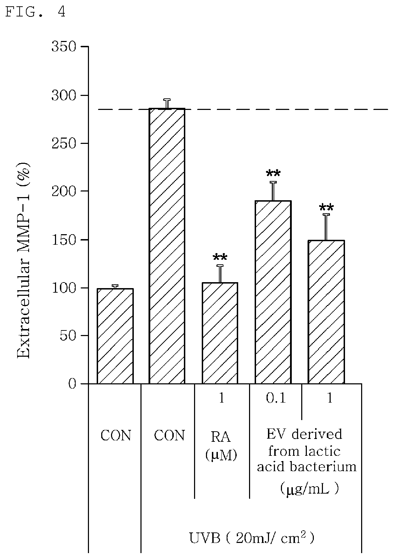

As shown in FIG. 4, the result verified that the extracellular vesicles derived from the lactic acid bacteria significantly decreased the expression of the extracellularly secreted MMP-1 enzyme and thus had an anti-aging effect. In addition, the result verified that the extracellular vesicles derived from the lactic acid bacteria repressed ultraviolet light-induced skin damage and thus had a skin-protecting effect against ultraviolet rays.

### Test Example 4. Comparison of Cytotoxicity

The effect of on cytotoxicity was compared between the extracellular vesicles derived from the lactic acid bacteria isolated in Example 1 and the lactic acid bacteria prepared in Comparative Example 1.

Specifically, human fibroblast Hs68 was cultured in a 5% CO₂ incubator at 37°C, and the extracellular vesicles derived from the lactic acid bacteria isolated in Example 1 and the lactic acid bacteria prepared in Comparative Example 1 were respectively incubated at 1-20 µg/ml for 72 hours. Then, the supernatants were collected, and LDH activity was measured. The cytotoxicity was measured by using the Pierce™ LDH Cytotoxicity Assay Kit according to the manufacturer's instructions.

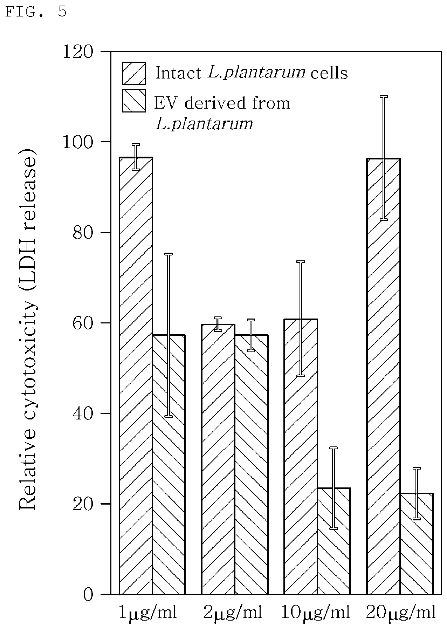

As shown in FIG. 5, the result showed that the extracellular vesicles derived from the lactic acid bacteria, when treated at a concentration higher than a specific concentration, significantly reduced the cytotoxicity in comparison with the case where the intact cells of lactic acid bacteria were used.

The examples the formulation of the composition according to one aspect of the present specification are described below, but various other formulations are also applicable. These examples are not intended to be limiting, but to illustrate specifically.

### [Formulation Example 1] Ointment

An ointment was prepared in a conventional manner according to the composition shown in Table 1 below.

**Table 1**

| Component | Content (wt. %) |
|---|---|
| Extracellular vesicles derived from the lactic acid bacteria of Example 1 | 2.00 |
| Glycerin | 8.00 |
| Butylene glycol | 4.00 |
| Liquid paraffin | 15.00 |
| Beta glucan | 7.00 |
| Carbomer | 0.10 |
| Caprylic/capric triglyceride | 3.00 |
| Squalane | 1.00 |
| Cetearyl glucoside | 1.50 |
| Sorbitan stearate | 0.40 |
| Cetearyl alcohol | 1.00 |
| Wax | 4.00 |
| Purified water | Balance |
| Sum | 100.00 |

### [Formulation Example 2] Cosmetic Solution Preparation

A cosmetic solution formulation was prepared in a conventional manner according to the composition shown in Table 2 below.

**Table 2**

| Component | Content (wt. %) |
|---|---|
| Extracellular vesicles derived from the lactic acid bacteria of Example 1 | 2.00 |
| Hydroxyethylene cellulose (2% aqueous solution) | 12.00 |
| Xanthan gum (2% aqueous solution) | 2.00 |
| 1,3-butylene glycol | 6.00 |
| Concentrated glycerin | 4.00 |
| Sodium hyaluronate (1% aqueous solution) | 5.00 |
| Purified water | Balance |
| Sum | 100.00 |

### [Formulation Example 3] Lotion

A lotion was prepared in a conventional manner according to the composition shown in Table 3 below.

**Table 3**

| Component | Content (wt. %) |
|---|---|
| Extracellular vesicles derived from the lactic acid bacteria of Example 1 | 2.00 |
| L-ascorbic acid-2-phosphate magnesium salt | 1. 00 |
| Water-soluble collagen (1% aqueous solution) | 1.00 |
| Sodium citrate | 0.10 |
| Citric acid | 0.05 |
| Licorice extract | 0.20 |
| 1,3-butylene glycol | 3.00 |
| Purified water | Balance |
| Sum | 100.00 |

### [Formulation Example 4] Cream

A cream was prepared in a conventional manner according to the composition shown in Table 4 below.

**Table 4**

| Component | Content (wt. %) |
|---|---|
| Extracellular vesicles derived from the lactic acid bacteria of Example 1 | 2.00 |
| Polyethylene glycol monostearate | 2.00 |
| Self-emulsifying monostearic acid glycerin | 5.00 |
| Cetyl alcohol | 4.00 |
| Squalene | 6.00 |
| Tri-2-ethylhexane glyceryl | 6.00 |
| Sphingoglycolipids | 1.00 |
| 1,3-butylene glycol | 7.00 |
| Purified water | Balance |
| Sum | 100.00 |

### [Formulation Example 5] Pack

A pack was prepared in a conventional manner according to the composition shown in Table 5 below.

**Table 5**

| Component | Content (wt. %) |
|---|---|
| Extracellular vesicles derived from the lactic acid bacteria of Example 1 | 2.00 |
| Polyvinyl alcohol | 13.00 |
| L-ascorbic acid-2-phosphate magnesium salt | 1.00 |
| Lauroyl hydroxyproline | 1.00 |
| Water-soluble collagen (1% aqueous solution) | 2.00 |
| 1,3-butylene glycol | 3.00 |
| Ethanol | 5.00 |
| Purified water | Balance |
| Sum | 100.00 |

So far, specific portions of the present disclosure have been described in detail. It will be obvious to one of ordinary skill in the art that this specific description is only a preferred embodiment and that the scope of the present disclosure is not limited thereby. Accordingly, the actual scope of the present disclosure will be defined by the appended claims and their equivalents.

## Claims

1. An anti-aging composition comprising as an active ingredient an extracellular vesicle derived from lactic acid bacteria.

2. The anti-aging composition of claim 1,
wherein the lactic acid bacteria are at least one selected from the group consisting of *Bifidobacterium* spp., *Latobacillus* spp., *Streptocuccus* spp., *Leuconostoc* spp., *Pediococcus* spp. and *Lactococcus* spp.

3. The anti-aging composition of claim 2,
wherein the lactic acid bacteria are a strain of *Lactobacillus* spp.

4. The anti-aging composition of claim 3,
wherein the strain of *Lactobacillus* spp. is *Lactobacillus plantarum.*

5. The anti-aging composition of claim 1,
wherein the extracellular vesicle is separated from a lactic acid bacteria culture solution.

6. The anti-aging composition of claim 1,
wherein the extracellular vesicle is an exosome-like vesicle.

7. The anti-aging composition of claim 6,
wherein the exosome-like vesicle has a diameter of 20 to 200 nm.

8. The anti-aging composition of claim 1,
wherein the extracellular vesicle is precipitated by ultracentrifugation of a lactic acid bacteria culture solution at 100,000 x g or higher.

9. The anti-aging composition of claim 1,
wherein the composition inhibits the expression of the MMP-1 enzyme.

10. The anti-aging composition of claim 1,
wherein the composition inhibits the expression of the MMP-1 enzyme induced by ultraviolet light

11. The anti-aging composition of claim 1,
wherein the composition has at least one of the following properties:
(a) improving skin wrinkles;
(b) increasing skin elasticity;
(c) suppressing collagen reduction; and
(d) preventing ultraviolet light-induced skin damage.

12. The anti-aging composition of claim 1,
wherein the composition is an external skin application composition.

13. The anti-aging composition of claim 1,
wherein the composition is a cosmetic composition.
